# FASCICULE DE BREVET EUROPEEN

(11) **EP 1 481 165 B1**
(45) Date de publication et mention de la délivrance du brevet: **27.07.2011**
(21) Numéro de dépôt: 03725253.3
(22) Date de dépôt: 24.02.2003
(51) Int. Cl.: F04B 17/00

(54) **ACTIONNEUR ET MOTEUR OSMOTIQUES**
OSMOSE-ANTRIEBSVORRICHTUNG
OSMOTIC ACTUATOR AND ENGINE

(30) Priorité: 28.02.2002 FR 0202558
(43) Date de publication de la demande: 01.12.2004
(73) Titulaire: UNIVERSITE JOSEPH FOURIER, 38041 Grenoble Cédex 9 (FR)
(72) Inventeur: CINQUIN Philippe, F-38700 la Tronche (FR); CINQUIN, Olivier, F-38700 La Tronche (FR)
(74) Mandataire: Thibon, Laurent
(86) Numéro de dépôt international: PCT/FR2003/000592
(87) Numéro de publication internationale: WO 2003/072941

(56) Documents cités:
- EP-A- 0 259 013
- EP-A- 0 354 742
- US-A- 3 894 538
- US-A- 5 279 608

## Description

La présente invention concerne des dispositifs pouvant servir d'actionneur ou de moteur qui sont simples à réaliser, qui utilisent un carburant à faible coût, et qui émettent peu ou pas de déchets.

En outre, la présente invention concerne des dispositifs pouvant servir d'actionneur ou de moteur adaptés à fonctionner à l'intérieur d'un milieu biologique tel que le corps humain ou un corps animal.

De tels actionneurs et de tels moteurs trouvent des applications dans le domaine médical par exemple pour pallier la déficience d'un muscle naturel. Les muscles pouvant être remplacés ou assistés, de façon temporaire ou définitive, sont, par exemple, le muscle cardiaque, les muscles respiratoires, les sphincters et les muscles lisses ou striés, en particulier squelettiques.

De tels actionneurs et de tels moteurs trouvent également des applications dans des domaines autres que médicaux. En particulier, un tel moteur peut être utilisé dans tous les domaines dans lesquels une faible production de déchets est un facteur important dans le choix du moteur. Il peut s'agir, par exemple, du domaine automobile où l'on cherche à réduire le plus possible les déchets polluants produits par le moteur utilisé pour l'entraînement des roues du véhicule.

La demande de brevet FR0109526, au nom de la demanderesse et non encore publiée, décrit un actionneur osmotique destiné à être plongé dans un milieu biologique et comprenant une enceinte déformable ayant une membrane semi-perméable, cette enceinte contenant un soluté susceptible d'être osmotiquement actif.

EP-A-0259013 divulgue un actionneur osmotique.

La présente invention vise à proposer un actionneur et un moteur osmotique pouvant fonctionner sur une longue durée et dont le fonctionnement peut être contrôlé avec davantage de précision.

Plus particulièrement, la présente invention prévoit un actionneur destiné à être disposé au contact d'un solvant biologique, comprenant une enceinte ayant une paroi perméable au solvant et non perméable à un premier soluté et contenant des microorganismes adaptés à transformer un second soluté en le premier soluté ; et une chambre déformable reliée à l'enceinte pouvant augmenter de volume sous l'action du solvant pénétrant dans l'enceinte par osmose.

Selon un mode de réalisation de l'invention, l'enceinte comprend un faisceau de fibres creuses colonisées par les microorganismes.

Selon un mode de réalisation de l'invention, l'enceinte a une paroi perméable au second soluté.

Selon un mode de réalisation de l'invention, l'enceinte a une paroi non perméable au second soluté, les microorganismes étant adaptés à transformer un nombre de particules du second soluté en un nombre plus élevé de particules du premier soluté.

La présente invention prévoit également un moteur comprenant un actionneur tel que précédemment décrit, dans lequel la chambre comporte un moyen de rappel qui s'oppose à l'augmentation du volume de la chambre et un moyen commandable pour faire baisser la pression osmotique dans la chambre. Selon un mode de réalisation de l'invention, le moteur comprend en outre une enceinte secondaire ayant une portion perméable au solvant et non perméable aux particules des premier et second solutés, et contient des microorganismes adaptés à transformer un nombre de particules du second soluté en un nombre plus faible de particules du premier soluté, ladite enceinte secondaire étant reliée à la chambre par une vanne.

Selon un mode de réalisation de l'invention, l'enceinte est disposée dans une enveloppe déformable contenant le solvant et le premier soluté, le moyen pour faire baisser la pression osmotique dans la chambre étant une vanne adaptée pour mettre en communication la chambre et l'enveloppe.

Selon un mode de réalisation de l'invention, l'enveloppe comprend des microorganismes adaptés à transformer un nombre de particules du second soluté en un nombre plus faible de particules du premier soluté.

Selon un mode de réalisation de l'invention, le moteur comprend un moyen de mise en communication de l'enveloppe avec une source d'apport de substances nécessaires au métabolisme des microorganismes.

Selon un mode de réalisation de l'invention, le moyen de mise en communication comprend un faisceau de fibres creuses traversant l'enveloppe et dans lesquelles peut circuler un fluide contenant lesdites substances

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
les figures 1A et 1B représentent deux étapes de fonctionnement d'un premier mode de réalisation d'un moteur selon l'invention ;
les figures 2A et 2B représentent deux étapes de fonctionnement d'une variante du premier mode de réalisation du moteur selon l'invention ;
les figures 3A à 3C représentent trois étapes de fonctionnement d'un deuxième mode de réalisation du moteur selon l'invention ; et
les figures 4A à 4D représentent quatre étapes de fonctionnement d'un troisième mode de réalisation du moteur selon l'invention.

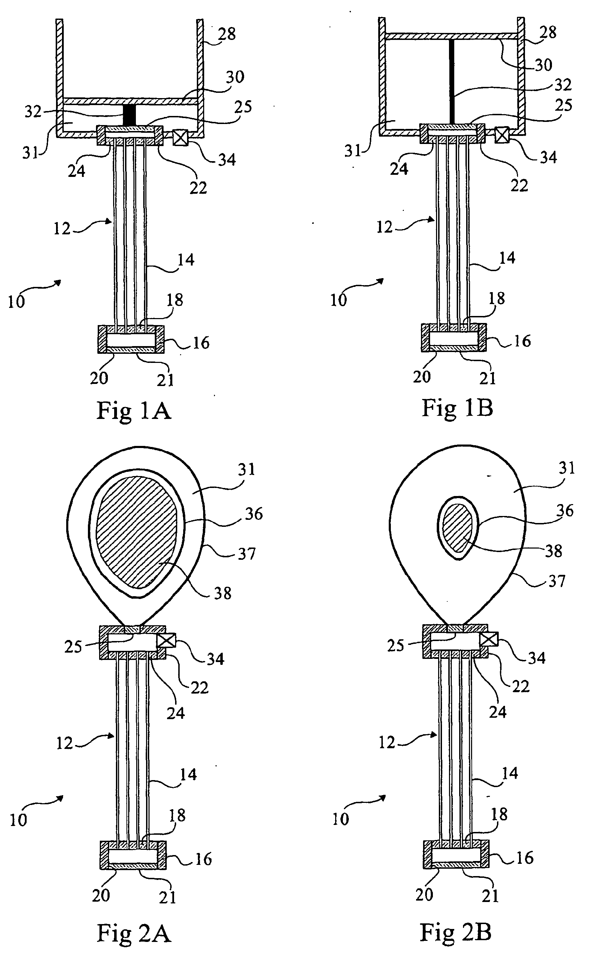

Sur les figures 1A et 1B, un moteur osmotique 10 selon l'invention comprend une enceinte 12 constituée par un faisceau de fibres creuses à paroi semi-perméables 14, par exemple du type utilisé pour les opérations de dialyse. La paroi des fibres 14 a un seuil de coupure de l'ordre de 200 Daltons, c'est-à-dire qu'elle laisse passer des particules ayant une masse molaire sensiblement inférieure à 200 Daltons, c'est-à-dire à 200 g/mol. Chaque fibre a, par exemple, un diamètre de l'ordre de 200 µm. Le faisceau de fibres 14 est maintenu à une première extrémité par une première bague de jonction 16, par exemple par l'intermédiaire d'une zone de collage 18. La bague 16 comprend une ouverture 20 fermée par un bouchon 21. La seconde extrémité du faisceau de fibres 14 est maintenue par une seconde bague de jonction 22, par exemple par l'intermédiaire d'une zone de collage 24. Une membrane 25, ayant un seuil de coupure de l'ordre de 1000 Daltons, ferme la seconde bague 22.

L'enceinte 12 est fixée au niveau de la seconde bague de jonction 22, sur une extrémité d'un corps cylindrique 28 dans lequel peut coulisser un piston mobile 30. Le piston mobile 30 et le corps cylindrique définissent une chambre de dilatation 31. Un moyen de rappel 32, par exemple un ressort, exerce sur le piston 30 un effort de traction tendant à le ramener dans une position de repos. Le corps cylindrique 28 comprend une vanne d'évacuation 34 communiquant avec l'extérieur du moteur 10.

On dispose à l'intérieur des fibres 14 une population de cellules bactériennes, végétales ou animales qui sont adaptées à fabriquer une substance X osmotiquement active. Le seuil de coupure des fibres 14 est fixé pour empêcher le passage de la substance X, tandis que le seuil de coupure de la membrane 25 est fixé pour permettre le passage de la substance X mais pour bloquer les cellules. Dans le présent exemple, la substance X est un di-hexose et les cellules peuvent consister en des cellules E. Coli génétiquement modifiées pour produire le di-hexose. Divers moyens de génie génétique permettent d'amener une cellule génétiquement modifiée à fabriquer du di-hexose et à l'exporter en dehors de la cellule.

Dans une phase initiale, pour disposer les cellules génétiquement modifiées à l'intérieur des fibres 14, on peut placer l'enceinte 12 seule dans un milieu ambiant osmotiquement neutre et faire circuler lentement à l'intérieur du faisceau de fibres 14, par l'intermédiaire de l'ouverture 20, un liquide de culture contenant les cellules génétiquement modifiées. Les seuils de coupure de la membrane 25 et de la paroi des fibres 14 sont suffisamment faibles pour retenir les cellules à l'intérieur des fibres 14. Le milieu ambiant comprend un solvant dans lequel sont dissous les substances nutritives essentielles au métabolisme des cellules, parmi lesquelles le glucose (dont la masse molaire est d'environ 180 Daltons) et l'oxygène. Le seuil de coupure des fibres 14 est déterminé pour laisser passer du milieu ambiant vers les cellules les substances nutritives et pour laisser passer, vers le milieu ambiant, les déchets produits par le métabolisme cellulaire, par exemple le gaz carbonique. Les cellules génétiquement modifiées peuvent ainsi coloniser l'intérieur des fibres 14. Les cellules sont par exemple traitées pour se déposer et adhérer sur la paroi interne des fibres 14 sous la forme d'une monocouche. Lorsque la colonisation est réalisée, l'ouverture 20 est fermée par le bouchon 21.

Les cellules peuvent, en outre, être traitées pour obtenir des propriétés susceptibles de faciliter le fonctionnement à long terme du moteur. On peut chercher à obtenir des cellules « immortalisées » pour favoriser le fonctionnement à long terme du dispositif. On peut chercher également à obtenir des cellules présentant une « inhibition de contact » de façon à mettre l'ensemble de la population cellulaire dans un état harmonieux permettant en particulier une bonne circulation des substances nutritives et cataboliques.

Un cycle de fonctionnement du moteur 10 se déroule de la façon suivante.

En fonctionnement normal, le moteur 10 est placé dans un milieu ambiant comprenant un solvant dans lequel sont dissoutes les substances nutritives essentielles au métabolisme des cellules, en particulier le glucose.

La figure 1A représente le moteur 10 au début d'un cycle. Le piston 30 est en position de repos, le volume de la chambre de dilatation 31 étant minimal et la vanne d'évacuation 34 est fermée. Les cellules génétiquement modifiées produisent, à partir du glucose, des molécules de di-hexose, ce qui tend à faire augmenter la pression osmotique à l'intérieur du faisceau de fibres 14. Le solvant du milieu ambiant pénètre dans les fibres 14 et dans la chambre de dilatation 31, déplaçant ainsi le piston 30. Le déplacement du piston 30 tend le ressort 32, permettant d'emmagasiner de l'énergie mécanique.

Sur la figure 1B, la chambre de dilatation 31 est représentée en expansion maximale. La vanne d'évacuation 34 s'ouvre alors. La pression à l'intérieur de la chambre de dilatation 31 s'égalise avec la pression du milieu ambiant. Le ressort 32 ramène le piston 30 en position de repos en évacuant, par la vanne d'évacuation 34, le solvant de la chambre de dilatation 31 dans le milieu ambiant. L'énergie mécanique emmagasinée dans le ressort 32 est ainsi récupérée. La vanne 34 est finalement fermée, achevant ainsi le cycle moteur.

Le piston 32 peut être relié à un élément extérieur auquel on souhaite transmettre une énergie mécanique.

Selon le premier mode de réalisation, la chambre de dilatation 31 est réalisée par un corps cylindrique dans lequel coulisse un piston. En fonction de l'utilisation souhaitée du moteur 10 selon l'invention, la chambre de dilatation 31 peut être réalisée de façon différente.

Les figures 2A et 2B représentent une autre variante de structure de la chambre de dilatation 31 du moteur 10 du premier mode de réalisation. Selon cette variante, la chambre de dilatation 31 correspond à l'espace défini entre une enveloppe intérieure 36 et une enveloppe extérieure 37 à la manière d'une chambre à air. L'enveloppe intérieure 36 est déformable et extensible et entoure un corps déformable 38. L'enveloppe extérieure 37 est souple et inextensible. Elle se referme sur l'enveloppe intérieure 36 et est reliée à la bague de jonction 22 de l'enceinte 12. La vanne d'évacuation 34 est disposée sur la bague de jonction 22. A titre d'exemple, dans une application médicale du moteur osmotique 10 selon l'invention, le corps déformable 38 peut être le coeur humain et les enveloppes peuvent définir des chambres de dilatation 31 en forme de boudins entourant le coeur.

Un cycle du moteur 10 selon la variante du premier mode de réalisation est le suivant.

La figure 2A représente le moteur 10 en début de cycle. Le volume de la chambre de dilatation 31 est minimal, le corps déformable 38 étant en dilation maximale, ce qui peut correspondre à un coeur en diastole. La vanne d'évacuation 34 est alors fermée. Les cellules génétiquement modifiées produisent du di-hexose, ce qui entraîne, par osmose, l'introduction de solvant dans la chambre de dilatation 31. L'enveloppe intérieure 36 se déforme et comprime le corps déformable 38.

Sur la figure 2B, le corps déformable 38 est comprimé au maximum, ce qui peut correspondre à un coeur en systole. A l'ouverture de la vanne d'évacuation 34, le solvant évacue la chambre de dilatation 31 permettant la dilation du corps déformable 38, ce qui achève le cycle.

Selon une autre variante de l'invention, la chambre de dilatation est constituée par une enveloppe élastique enfermant les fibres qui sont disposées, par exemple, en spirale, les deux bagues de jonction étant étanches. Lorsque les cellules produisent la substance osmotiquement active, les fibres ont tendance à se redresser et à déformer l'enveloppe élastique. On prévoit une vanne d'évacuation au niveau d'une bague de jonction. A l'ouverture de la vanne, la pression à l'intérieur des fibres diminue et l'enveloppe tend à reprendre sa forme initiale.

Selon une autre variante de l'invention, l'enceinte peut être reliée à la chambre de dilatation par un conduit souple. Ceci permet de disposer de façon avantageuse l'enceinte dans un milieu ambiant propice pour l'apport en solvant et en glucose, et de placer la chambre de dilatation à un endroit où l'on souhaite disposer de l'énergie mécanique. Dans le cas d'une application médicale, on pourra disposer l'enceinte dans un tissu graisseux, ou sur le réseau vasculaire. Dans ce dernier cas, les fibres peuvent être agencées pour former un tube creux, laissant en son centre un espace cylindrique permettant la circulation d'un fluide tel que le sang. Les bagues de jonction peuvent être de forme torique et placées contre la paroi d'un vaisseau sanguin. L'une des bagues de jonction toriques communique avec la chambre de dilatation par le conduit souple qui perfore le vaisseau sanguin.

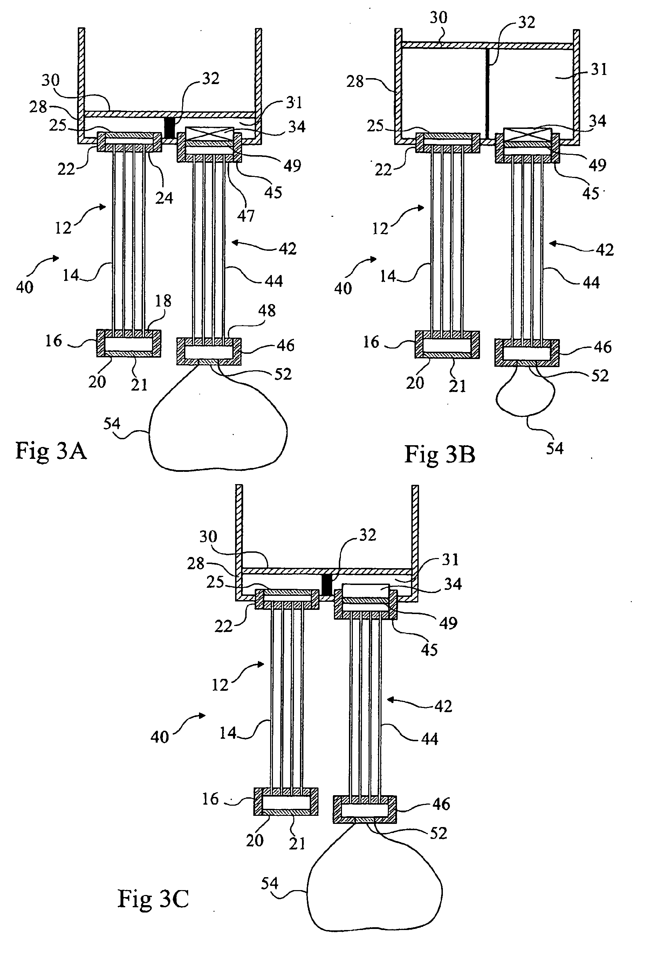

Les figures 3A à 3C représentent un deuxième mode de réalisation d'un moteur osmotique selon l'invention. Le moteur 40 inclut les composants du moteur du premier mode de réalisation et les références associées à celui-ci sont conservées.

Le moteur 40 comprend une première enceinte 12 du type décrit précédemment et une second enceinte 42. La seconde enceinte 42 comprend un second faisceau de fibres 44 maintenu aux extrémités par des bagues de jonction 45, 46 au moyen de zones de collage 47, 48. La seconde enceinte 42 est fixée sur le corps cylindrique 28 au niveau de la vanne 34, par la bague de jonction 45 qui comprend une membrane 49 séparant la chambre de dilatation 31 des secondes fibres 44. La seconde enceinte 42 communique, au niveau de la bague 46 par l'intermédiaire d'une membrane 52, avec un réservoir 54 déformable et étanche.

Le premier faisceau de fibres 14 est colonisé par une première population P₁ de cellules génétiquement modifiées produisant une substance X (par exemple du lactose) à partir d'une substance Y (par exemple du di-lactose), de sorte qu'à partir d'une particule élémentaire de la substance Y, il est produit plus d'une particule élémentaire de la substance X. Le second faisceau de fibres 44 est colonisé par une seconde population P₂ de cellules génétiquement modifiées produisant la substance Y à partir de la substance X, de sorte que pour produire une particule élémentaire de la substance Y, il est utilisé plus d'une particule élémentaire de la substance X.

Les parois des faisceaux de fibres 14, 44 ont un seuil de coupure inférieur à la masse molaire des substances X et Y. A titre d'exemple, le seuil de coupure est de l'ordre de 200 Daltons lorsque la substance X est du lactose et la substance Y du di-lactose. Les membranes 25, 49 et 52 ont des seuils de coupure supérieurs à 1000 Daltons, de façon à laisser passer les substances X et Y et maintenir les cellules génétiquement modifiées dans les faisceaux de fibres respectifs 14, 44.

En fonctionnement normal, le moteur 40 est placé dans un milieu ambiant comprenant un solvant dans lequel sont dissoutes les substances nutritives essentielles au métabolisme des cellules, en particulier le glucose. Le cycle de fonctionnement du moteur osmotique 40 selon l'invention est le suivant.

La figure 3A représente le moteur 40 au début du cycle. La vanne 34 est fermée. Les concentrations de la substance X sont identiques dans le réservoir 54 et la chambre de dilatation 31, de même que les concentrations de la substance Y, et en glucose. Dans le premier faisceau de fibres 14, la population P₁ produit de la substance X, ce qui augmente la pression osmotique dans la chambre de dilatation 31. Le solvant du milieu ambiant pénètre dans le premier faisceau de fibres 14 puis dans la chambre de dilatation 31, déplaçant ainsi le piston 30 et emmagasinant de l'énergie mécanique par la mise en tension du ressort 32. Pendant ce temps, dans le second faisceau de fibres 44, la population P₂ produit de la substance Y, ce qui diminue la pression osmotique dans le réservoir 54. Le réservoir 54 diminue de volume, sans produire de travail utile puisque rien ne s'oppose à cette diminution.

La figure 3B représente le moteur 10 à la fin de l'étape décrite précédemment, la chambre de dilatation 31 ayant un volume maximum.

La vanne 34 s'ouvre alors. Les concentrations en substance X et en substance Y s'équilibrent dans les faisceaux de fibres 14, 44, la chambre de dilatation 31 et le réservoir 54. De même, les pressions osmotiques s'équilibrent dans les différents compartiments. Le piston 30 descend donc sous l'action du ressort 32 jusqu'à la position représentée sur la figure 3C. De plus, le réservoir 54 se dilate en se remplissant de liquide, le travail nécessaire pour dilater le réservoir 54 étant négligeable devant celui procuré par le ressort 32, les pressions dans le milieu ambiant étant faibles devant celles présentes dans la chambre de dilatation 31. La vanne 34 est alors refermée, ce qui clôt le cycle.

Dans le deuxième mode de réalisation, les parois des fibres 14, 44 ne laissent passer que le solvant du milieu ambiant ainsi que les substances intervenant dans le métabolisme des cellules génétiquement modifiées, notamment le glucose, l'oxygène ou le dioxyde de carbone.

Le deuxième mode de réalisation est particulièrement avantageux dans la mesure où les échanges entre le moteur 40 et le milieu ambiant sont réduits par rapport au premier mode de réalisation. En effet, dans le premier mode de réalisation, la substance osmotiquement active, le di-hexose, est produite à partir de glucose présent dans le milieu ambiant. En outre, à la fin d'un cycle moteur, la vanne d'évacuation 34 est ouverte et la majeure partie du di-hexose produit par les cellules est libérée dans le milieu ambiant. Dans le cas d'une application médicale, le di-hexose produit est libéré dans le corps humain, ce qui peut être problématique. Dans le second mode de réalisation, il y a bien une consommation de glucose par les cellules, mais uniquement pour leur métabolisme normal, c'est-à-dire dans une proportion très inférieure à celle du premier mode de réalisation.

Selon une variante du deuxième mode de réalisation, la substance X peut être du glucose et la substance Y du lactose. Le seuil de coupure des membranes des fibres 14, 44 est alors fixé à un seuil inférieur à celui du glucose, par exemple 100 Daltons. Le glucose nécessaire au métabolisme des cellules ne peut donc plus traverser ces membranes. Le réservoir 54 comprend alors un moyen pour le mettre en communication avec le milieu ambiant. Il peut s'agir, par exemple, d'une vanne associée à une membrane ayant un seuil de coupure à 200 Daltons. La vanne est par exemple ouverte pendant un court laps de temps lorsque le réservoir est à un volume minimum. Ceci permet à des molécules de glucose du milieu ambiant de pénétrer dans le réservoir. Ces molécules de glucose permettront de compenser les pertes liées au métabolisme cellulaire.

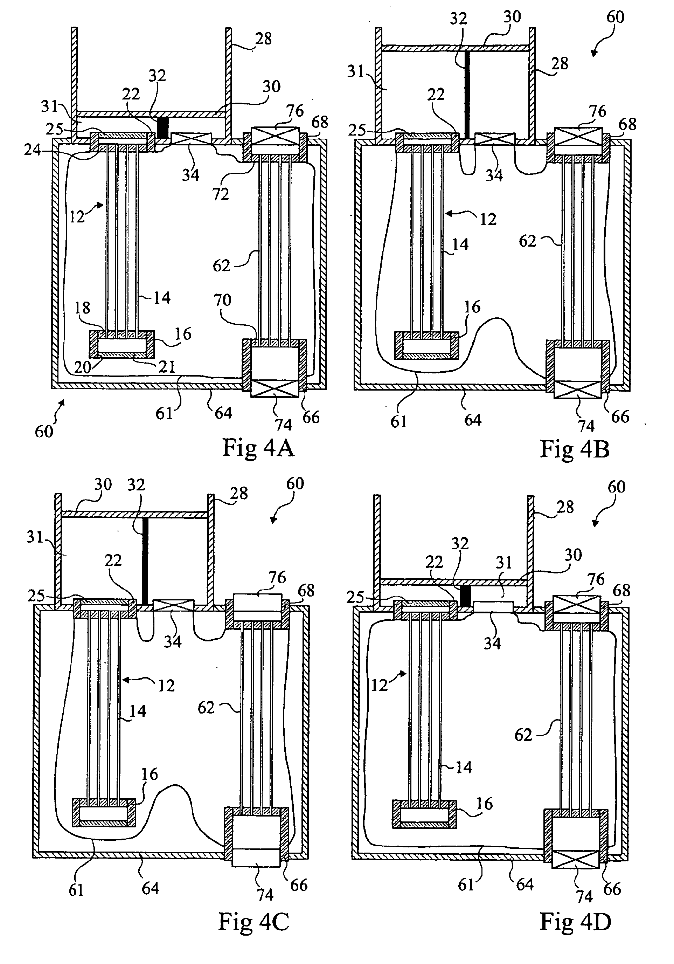

Les figures 4A à 4D représentent un troisième mode de réalisation du moteur osmotique 60 selon l'invention. Le moteur 60 inclut les composants du moteur 10 du premier mode de réalisation et les références associées à celui-ci sont conservées.

L'enceinte 12 est disposée dans une enveloppe 61 déformable et étanche qui se referme sur la bague de jonction 22 et la vanne d'évacuation 34. L'enceinte est remplie d'un liquide biologique. Un second faisceau de fibres 62 est disposé dans l'enveloppe 61. L'enveloppe 61 peut être disposée dans un carter rigide 64 perforé pour ne pas empêcher les déformations de l'enveloppe 61. Le faisceau de fibres 62 est maintenu à ses extrémités par des bagues de jonction 66, 68, par exemple par des zones de collage 70, 72. Les bagues de jonction 66, 68 traversent l'enveloppe 61 et sont fixées sur le carter rigide 64. Elles comportent chacune une vanne 74, 76 permettant de mettre en communication l'espace interne des fibres 62 avec l'extérieur du carter 64.

Le premier faisceau de fibres 14 est colonisé par une première population P₁ de cellules génétiquement modifiées produisant une substance X (par exemple du di-hexose) à partir d'une substance Y (par exemple du quadri-hexose), de sorte qu'à partir d'une particule élémentaire de Y, on produit plus d'une particule élémentaire de X. Une seconde population P₂ de cellules génétiquement modifiées, disposées à l'intérieur de l'enveloppe 61, est adaptée à produire la substance Y à partir de la substance X, de sorte que pour produire une particule élémentaire de la substance Y, on utilise plus d'une particule élémentaire de la substance X. La seconde population P₂ de cellules peut être déposée sur les parois extérieures des fibres 62.

Les membranes des faisceaux de fibres 14, 62 ont un seuil de coupure supérieur à la masse molaire du glucose mais inférieur à la masse molaire des substances X et Y. A titre d'exemple, le seuil de coupure est de l'ordre de 200 Daltons lorsque la substance X est un di-hexose, et la substance Y un quadri-hexose.

Le carter rigide 64 peut être disposé dans un milieu biologique de sorte qu'un fluide biologique puisse s'écouler dans le second faisceau de fibres 62 lorsque les vannes 74, 76 sont ouvertes. On peut également connecter directement des conduites d'alimentation et d'évacuation en fluide au niveau des bagues de jonction 66, 68. Le seuil de coupure, par exemple de 100 Daltons, de la membrane du faisceau de fibres 62 permet le passage des substances nécessaires au métabolisme des populations P₁, P₂ des cellules.

Un cycle de fonctionnement du moteur 60 selon le troisième mode de réalisation est le suivant.

La figure 4A représente le moteur 60 au début d'un cycle. Les vannes 34, 74, 76 sont fermées. L'enveloppe 61 est au maximum de son volume. Tous les compartiments contiennent un solvant où sont en solution les substances X et Y. La première population P₁ de cellules commence à produire de la substance X, ce qui augmente la pression osmotique dans le faisceau 14 et la chambre de dilatation 31. La seconde population P₂ commence à produire de la substance Y, réduisant la pression osmotique à l'intérieur de l'enveloppe 61. Les échanges de liquide se produisent, le liquide passant vers le premier faisceau de fibres 14 et, de là, vers la chambre de dilatation 31 entraînant le déplacement du piston 30. Le piston est en phase ascendante.

La figure 4B représente le moteur 60 à la fin de l'étape précédemment décrite. La concentration en substance Y est minimale dans le premier faisceau de fibres 14 et maximale à l'intérieur de l'enveloppe 61. A l'inverse, la concentration en substance X est maximale dans le premier faisceau de fibres 14 et minimale dans l'enveloppe 61. Les différents compartiments ont une concentration en oxygène réduite par rapport au début du cycle et une concentration en dioxyde de carbone supérieure à celle du début du cycle.

En figure 4C, on ouvre les vannes 74, 76 de façon à mettre en communication le second faisceau de fibres 62 avec un fluide extérieur au carter rigide 64. Les concentrations gazeuses s'équilibrent alors dans l'ensemble des compartiments. De même, les concentrations en glucose s'équilibrent entre l'enveloppe 61 et le fluide extérieur sans que la position du piston varie.

On ferme ensuite les vannes 74, 76 et on ouvre la vanne d'évacuation 34 qui met en communication directe la chambre de dilatation 31 et l'enveloppe 61. La pression dans la chambre de dilatation 31 s'effondre et le ressort de rappel 32 ramène le piston 30 en position initiale évacuant le solvant de la chambre de dilatation 31 vers l'enveloppe 61. Le piston est dit en phase descendante. Le premier faisceau de fibres 14 est ainsi mis en communication avec l'intérieur de l'enveloppe 61. Les concentrations en substances X et Y s'égalisent entre ces deux compartiments.

La figure 4D représente le moteur 60 à la fin de la phase descendante du piston 30. La vanne 64 est ensuite fermée, ce qui clôt le cycle.

Une variante du quatrième mode de réalisation du moteur osmotique peut être utilisée comme moteur pour l'entraînement des roues d'un véhicule automobile. Selon cette variante, le ressort est supprimé et le piston est relié, par exemple, par une bielle à un arbre moteur d'entraînement des roues de façon analogue à la liaison entre un piston d'un moteur thermique et le vilebrequin. La force motrice correspond à la phase ascendante du piston, c'est-à-dire à la phase d'expansion de la chambre de dilatation. En phase descendante, lorsque le volume de la chambre de dilatation diminue, le piston ne rencontre qu'une faible résistance, correspondant au passage du solvant au travers de la vanne d'évacuation de la chambre de dilatation. De façon avantageuse, au moins deux moteurs osmotiques peuvent être mis en parallèle pour entraîner l'arbre moteur de sorte que les chambres de dilatation des moteurs travaillent en opposition, l'une étant en phase d'expansion lorsque l'autre est en phase de contraction.

Un tel moteur peut, en outre, être utilisé pour récupérer de l'énergie lors du freinage du véhicule. Dans ce cas, il comporte une vanne supplémentaire, appelée vanne d'alimentation disposée entre le premier faisceau de fibres et la chambre de dilatation. Lors du fonctionnement du moteur pour l'entraînement des roues, la vanne d'alimentation est ouverte de sorte que le moteur fonctionne comme cela a été précédemment décrit.

Dans le cas d'un freinage, quand le piston est en phase ascendante, la vanne d'alimentation est fermée et la vanne d'évacuation est ouverte de sorte que la chambre de dilatation se remplit de liquide sans effort significatif. Une grande partie du liquide contenue dans l'enveloppe déformable passe dans la chambre de dilatation sans que les concentrations dans les divers compartiments varient. La vanne d'alimentation est ensuite ouverte et la vanne d'évacuation fermée. Lorsque le piston descend sous l'action de l'arbre moteur entraîné par les roues, le liquide contenu dans la chambre de dilatation est chassé au travers du faisceau de fibres et arrive dans l'enveloppe. Le travail ainsi produit permet à la fois de ralentir le véhicule, mais également de faire varier les concentrations des substances X et Y. En effet, l'osmolarité du premier faisceau de fibres augmente, alors que l'osmolarité dans l'enveloppe déformable diminue.

De ce fait, lorsque le moteur fonctionne à nouveau comme moteur d'entraînement des roues, la vanne d'alimentation étant ouverte, le cycle moteur reprend avec une efficacité supérieure. En effet, les différences de concentrations entre les compartiments vont créer une différence de pression grâce à laquelle le cycle se réalisera plus rapidement.

Bien entendu, la présente invention est susceptible de diverses variantes et modifications qui apparaîtront à l'homme de l'art. En particulier, pour les deuxième et troisième modes de réalisation du moteur, la chambre de dilatation peut être réalisée selon les variantes décrites du premier mode de réalisation.

En outre, chaque enceinte 12, 42, 62 peut être réalisée autrement que par un faisceau de fibres. Elle peut avoir toute forme permettant un bon échange des solutés et du solvant de part et d'autre de la paroi de l'enceinte et facilitant la colonisation de l'enceinte par une population de cellules génétiquement modifiées.

De plus, chaque vanne 34, 74, 76 peut présenter tout type de structure connue. Les ouvertures et fermetures de chaque vanne peuvent par exemple être commandées par un dispositif externe au moteur, de façon synchrone ou non, ou bien être déclenchées automatiquement par la structure même de la vanne lorsque la pression dans un compartiment du moteur excède une valeur déterminée.

## Revendications

1. Actionneur, destiné à être disposé au contact d'un solvant biologique, comprenant :
- une enceinte (12) ayant une paroi perméable au solvant et non perméable à un premier soluté et contenant des microorganismes adaptés à transformer un second soluté en le premier soluté ; et
- une chambre déformable (31) reliée à l'enceinte, pouvant augmenter de volume sous l'action du solvant pénétrant dans l'enceinte par osmose.

2. Actionneur selon la revendication 1, **caractérisé en ce que** l'enceinte (12) comprend un faisceau de fibres creuses colonisées par les microorganismes.

3. Actionneur selon la revendication 1, dans lequel l'enceinte (12) a une paroi perméable au second soluté.

4. Actionneur selon la revendication 1, dans lequel l'enceinte (12) a une paroi non perméable au second soluté, les microorganismes étant adaptés à transformer un nombre de particules du second soluté en un nombre plus élevé de particules du premier soluté.

5. Moteur (10, 40, 60) comprenant un actionneur selon la revendication 1, dans lequel la chambre (31) comporte un moyen de rappel (32) qui s'oppose à l'augmentation du volume de la chambre et un moyen commandable (34) pour faire baisser la pression osmotique dans la chambre.

6. Moteur (40) comprenant un actionneur selon la revendication 4, et comprenant en outre une enceinte secondaire (42) ayant une portion perméable au solvant et non perméable aux particules des premier et second solutés et contenant des microorganismes adaptés à transformer un nombre de particules du second soluté en un nombre plus faible de particules du premier soluté, ladite enceinte secondaire étant reliée à la chambre (61) par une vanne (34).

7. Moteur (60) selon la revendication 5 ou 6, dans lequel l'enceinte (12) est disposée dans une enveloppe déformable (61) contenant le solvant et le premier soluté, le moyen (34) pour faire baisser la pression osmotique dans la chambre étant une vanne (34) adaptée pour mettre en communication la chambre (31) et l'enveloppe.

8. Moteur (60) selon la revendication 7, dans lequel l'enveloppe (61) comprend des microorganismes adaptés à transformer un nombre de particules du second soluté en un nombre plus faible de particules du premier soluté.

9. Moteur (60) selon la revendication 8, comprenant un moyen de mise en communication (62) de l'enveloppe (61) avec une source d'apport de substances nécessaires au métabolisme des microorganismes.

10. Moteur (60) selon la revendication 9, dans lequel le moyen (62) de mise en communication comprend un faisceau de fibres creuses traversant l'enveloppe (61) et dans lesquelles peut circuler un fluide contenant lesdites substances.

## Claims

1. An actuator to be arranged in contact with a biological solvent, comprising:
- an enclosure (12) having a wall permeable to the solvent and non-permeable to a first solute and containing microorganisms capable of transforming a second solute into the first solute; and
- a deformable chamber (31) connected to the enclosure that can see its volume increase under the action of the solvent penetrating into the enclosure by osmosis.

2. The actuator of claim 1, wherein the enclosure (12) comprises a bundle of hollow fibers colonized by the microorganisms.

3. The actuator of claim 1, wherein the enclosure (12) has a wall permeable to the second solute.

4. The actuator of claim 1, wherein the enclosure (12) has a wall non-permeable to the second solute, the microorganisms being capable of transforming a number of particles of the second solute into a higher number of particles of the first solute.

5. A motor (10, 40, 60) comprising the actuator of claim 1, wherein the chamber (31) comprises a return means (32) which opposes to the volume increase of the chamber and a controllable means (34) for decreasing the osmotic pressure in the chamber.

6. A motor (40) comprising the actuator of claim 4, and further comprising a secondary enclosure (42) having a portion permeable to the solvent and non-permeable to the particles of the first and second solutes, and containing microorganisms capable of transforming a number of particles of the second solute into a smaller number of particles of the first solute, said secondary enclosure being connected to the chamber (61) by a valve (34).

7. The motor (60) of claim 5 or 6, wherein the enclosure (12) is arranged in a deformable envelope (61) containing the solvent and the first solute, the means (34) for decreasing the osmotic pressure in the chamber being a valve (34) capable of permitting communication between the chamber (31) and the envelope.

8. The motor (60) of claim 7, wherein the envelope (61) comprises microorganisms capable of transforming a number of particles of the second solute into a smaller number of particles of the first solute.

9. The motor (60) of claim 8, comprising a means (62) for permitting communication between the envelope (61) and a source for supplying substances necessary to the metabolism of the microorganisms.

10. The motor (60) of claim 9, wherein the communication means (62) comprises a bundle of hollow fibers crossing the envelope (61) and in which a fluid containing said substances can circulate.

## Patentansprüche

1. Betätigungsvorrichtung zur Anordnung in Kontakt mit einem biologischen Lösungsmittel, welche Folgendes aufweist:
eine Umhüllung (12) mit einer Wand, die für das Lösungsmittel durchlässig ist und nicht für einen ersten gelösten Stoff durchlässig ist und Mikroorganismen enthält, die einen zweiten gelösten Stoff in den ersten gelösten Stoff umwandeln können; und
eine verformbare Kammer (31), die mit der Umhüllung verbunden ist, die ihr Volumen unter der Wirkung des Lösungsmittels vergrößern kann, welches durch Osmose in die Umhüllung eindringt.

2. Betätigungsvorrichtung nach Anspruch 1, wobei die Umhüllung (12) ein Bündel von hohlen Fasern aufweist, die von den Mikroorganismen besiedelt sind.

3. Betätigungsvorrichtung nach Anspruch 1, wobei die Umhüllung (12) eine Wand besitzt, die für den zweiten gelösten Stoff durchlässig ist.

4. Betätigungsvorrichtung nach Anspruch 1, wobei die Umhüllung (12) eine Wand besitzt, die nicht für den zweiten gelösten Stoff durchlässig ist, wobei die Mikroorganismen eine Anzahl von Partikeln des zweiten gelösten Stoffes in eine höhere Anzahl von Partikeln des ersten gelösten Stoffes umwandeln können.

5. Motor (10, 40, 60), der die Betätigungsvorrichtung nach Anspruch 1 aufweist, wobei die Kammer (31) Rückleitungsmittel (32) aufweist, die der Volumenzunahme der Kammer entgegenwirken, und steuerbare Mittel (34) zum Verringern des osmotischen Druckes in der Kammer.

6. Motor (40), der die Betätigungsvorrichtung nach Anspruch 4 aufweist und weiter eine zweite Umhüllung (42) aufweist, die einen Teil besitzt, der für das Lösungsmittel durchlässig ist und nicht für die Partikel der ersten und zweiten gelösten Stoffe durchlässig ist, und die Mikroorganismen enthält, die eine Anzahl von Partikeln des zweiten gelösten Stoffes in eine kleinere Anzahl von Partikeln des ersten gelösten Stoffes umwandeln können, wobei die zweite Umhüllung mit der Kammer (61) durch ein Ventil (34) verbunden ist.

7. Motor (60) nach Anspruch 5 oder 6, wobei die Umhüllung (12) in einer verformbaren Hülle (61) angeordnet ist, die das Lösungsmittel und den ersten gelösten Stoff enthält, wobei die Mittel (34) zum Verringern des osmotischen Druckes in der Kammer ein Ventil (34) sind, welches eine Verbindung zwischen der Kammer (31) und der Hülle gestattet.

8. Motor (60) nach Anspruch 7, wobei die Hülle (61) Mikroorganismen aufweist, die eine Anzahl von Partikeln des zweiten gelösten Stoffes in eine kleinere Anzahl von Partikeln des ersten gelösten Stoffes umwandeln können.

9. Motor (60) nach Anspruch 8, der Mittel (62) aufweist, um eine Verbindung zwischen der Hülle (61) und einer Quelle zum Liefern von Substanzen gestattet, die für den Metabolismus der Mikroorganismen nötig sind.

10. Motor (60) nach Anspruch 9, wobei die Verbindungsmittel (62) ein Bündel von hohlen Fasern aufweisen, welche durch die Hülle (61) verlaufen, und in denen ein Strömungsmittel zirkulieren kann, welches die Substanzen enthält.
